# EUROPÄISCHE PATENTANMELDUNG

(11) **EP 0 805 140 A1**
(43) Veröffentlichungstag der Anmeldung: **05.11.1997**
(21) Anmeldenummer: 97106679.0
(22) Anmeldetag: 23.04.1997
(51) Int. Cl.: C07C 63/70, C07C 51/29, C07C 49/80

(54) **Verfahren zur Herstellung von 4,5-Dichlor-2-methylbenzoesäure**

(30) Priorität: 02.05.1996 DE 19617558
(71) Anmelder: HOECHST AKTIENGESELLSCHAFT, 65929 Frankfurt am Main (DE)
(72) Erfinder: Wessel, Thomas, Dr., 60386 Frankfurt (DE); Koch, Peter, Dr., 63179 Obertshausen (DE)

(57) **Zusammenfassung**

Die vorliegende Erfindung betrifft ein Verfahren zur Herstellung von 4,5-Dichlor-2-methylbenzoesäure sowie deren Salze in hohen Ausbeuten und mit hohen Reingehalten durch Umsetzung von 3,4-Dichlortoluol mit Acetylchlorid oder Chloracetylchlorid in Gegenwart eines Friedel-Crafts-Katalysators und anschließende Oxidation der acetylierten Zwischenstufe zur Carbonsäure.

## Beschreibung

Die vorliegende Erfindung betrifft ein Verfahren zur Herstellung von 4,5-Dichlor-2-methylbenzoesäure sowie deren Salze in hohen Ausbeuten und mit hohen Reingehalten durch Umsetzung von 3,4-Dichlortoluol mit Acetylchlorid oder Chloracetylchlorid in Gegenwart eines Friedel-Crafts-Katalysators und anschließende Oxidation der acetylierten Zwischenstufe zur Carbonsäure.

4,5-Dichlor-2-methylbenzoesäure und die acetylierten Vorstufen stellen, wie viele ortho-substituierte aromatische Carbonsäurederivate, wichtige Zwischenprodukte für die Herstellung von Pharmazeutika und Pflanzenschutzmitteln dar.

Es ist bekannt 4,5-Dichlor-2-methylacetophenon durch Umsetzung von 2 mol 3,4-Dichlortoluol mit 2 mol Acetylchlorid und Aluminiumchlorid bei 100 °C herzustellen (Lutz et al., J. Org. Chem. 1947, 12, 617-686 bzw. DE-A 2142564). Hiernach wird die Acetylverbindung allerdings erst nach zweimaliger Umkristallisation aus Ethanol annähernd isomerenrein in 60 % Ausbeute gewonnen. Chloracetylierungen des 3,4-Dichlortoluols sind bislang nicht untersucht.
4,5-Dichlor-2-methylbenzoesäure ist in US 3,236,623 über eine Chlorierung von Dichlor-orthoxylol, anschließende Reaktion mit Natriumformiat zum Methyl-dichlorbenzyl-formiat und Salpetersäure-Oxidation im Gemisch mit 3,4-Dichlor-2-methylbenzoesäure beschrieben, wobei die Reinigung über fraktionierte Kristallisation aus Benzol erfolgt.

Angesichts dieses Standes der Technik ist es Aufgabe der vorliegenden Erfindung, ein unter ökologisch und arbeitshygienischen Aspekten verbessertes, die Abwasserbelastung verringerndes und die Verwendung von toxikologisch bedenklichen Lösungsmitteln vermeidendes Verfahren zur Herstellung von 4,5-Dichlor-2-methylbenzoesäure bereitzustellen, das in technisch einfacher Weise in hoher Ausbeute hochreine Produkte liefert.

Die vorliegende Erfindung betrifft ein Verfahren zur Herstellung von 4,5-Dichlor-2-methylbenzoesäure oder deren Salze der allgemeinen Formel I, 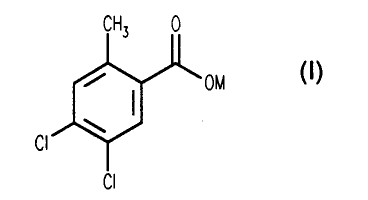 worin M für Wasserstoff oder ein Äquivalent eines Alkali- oder Erdalkalimetalls steht, dadurch gekennzeichnet, daß man 3,4-Dichlortoluol in Gegenwart eines Friedel-Crafts-Katalysators acetyliert oder Halogen-acetyliert, das erhaltene Isomerengemisch in einer Haloformreaktion in Gegenwart eines Alkali- oder Erdalkalihydroxids oxidiert, anschließend die erhaltene 4,5-Dichlor-2-methylbenzoesäure in Form eines ihrer Alkali- oder Erdalkalisalzes reinigt und gegebenenfalls das Salz in die freie Saure überführt.

Als Acetylierungsmittel werden bevorzugt Acetylchlorid, Chloracetylchlorid oder Acetanhydrid eingesetzt.

Als Friedel-Crafts-Katalysatoren eignen sich besonders Aluminiumchlorid, Zinkchlorid und Eisen(III)chlorid, wobei Aluminiumchlorid besonders bevorzugt ist.

Üblicherweise wird gemäß erfindungsgemäßem Verfahren der Friedel-Crafts-Katalysator in überschüssigem 3,4-Dichlortoluol vorgelegt und das Acetylierungsmittel eindosiert. Das Eindosieren des Friedel-Crafts-Katalysators bei vorgelegtem Acetylierungsmittel in 3,4-Dichlortoluol ist aber auch möglich.

Nach Reaktionsende, Hydrolyse und Phasentrennung des Reaktionsansatzes wird überschüssiges 3,4-Dichlortoluol durch Destillation zurückgewonnen und kann einem Folgeansatz wieder zugesetzt werden. Vakuumdestillation oder Wasserdampfdestillation ohne vorherige Phasentrennung stellen hierbei die bevorzugten Ausführungsformen dar.

Auch das acetylierte oder chloracetylierte Zwischenprodukt kann über Destillation im Vakuum gewonnen werden. Eine weitere bevorzugte Isolierungsmöglichkeit, insbesondere bei Chloracetylierungen, stellt die Filtration dar, nachdem 3,4-Dichlortoluol zuvor über Wasserdampfdestillation ausgekreist und das Produkt durch Abkühlen unter Rührung granuliert wurde.

Die Reaktion von 3,4-Dichlortoluol mit Acetylchlorid, Chloracetylchlorid oder Acetanhydrid wird bevorzugt bei Temperaturen von 10 bis 90 °C, besonders bevorzugt 20 bis 70 °C und ganz besonders bevorzugt bei 30 bis 50 °C durchgeführt.

Die anschließende Haloformoxidation wird bevorzugt direkt mit dem erhaltenen Isomerengemisch der Friedel-Crafts-Acetylierung duchgeführt. Als Alkali- und Erdalkalihydroxide kommen beispielsweise Lithium-, Natrium-, Kalium-, Rubidium-, Cäsium-, Magnesium-, Calcium-, Strontium- und Bariumhydroxid in Betracht. Bevorzugte Alkalimetallhydroxide sind Natrium- und Kalium-, ganz besonders bevorzugt Natriumhydroxid.

Als Oxidtionsmittel dienen Hypohalogenite bzw. Halogene in alkalischer Lösung z.B. Hypochlorit, Hypobromit oder Hypoiodit, wobei Hypochlorit und Hypobromit bevorzugt sind. Ganz besonders bevorzugt ist Hypochlorit in Form der kommerziell verfügbaren Chlorbleichlauge, hierbei wiederum ganz besonders bevorzugt in Konzentrationen mit ca. 50 g bis 200 g aktivem Chlor pro Liter Lösung.

Die Reihenfolge des Zusammengebens der Reaktionspartner ist bei der Oxidation variabel. Es kann beispielsweise acetylierte oder chloracetylierte Zwischenstufe in das Oxidationmittel eindosiert werden. Bevorzugt ist allerdings, Oxidationsmittel zum vorgelegten Acetophenon zu dosieren. Wird Chlorbleichlauge als Oxidationsmittel der Haloformreaktion eingesetzt, so kann eine 1.5fache bis 4fache molare Menge an aktivem Chlor, bezogen auf die Acetyl- oder Chloracetylverbindung, zweckmäßig sein.
Die acetylierten Zwischenstufen werden bevorzugt mit 2.8 bis 3.5facher molarer Menge an aktivem Chlor oxidiert, chloracetylierte Zwischenstufen werden dagegen bevorzugt mit der 1.7 bis 2.4facher Menge an aktivem Chlor umgesetzt.
Die Oxidationsstufe des erfindungsgemäßen Verfahrens kann je nach der Ausführungsart in der Kälte oder in der Hitze erfolgen. Bevorzugt wird die Umsetzung zwischen 0 °C und 100 °C durchgeführt. Besonders bevorzugt sind Temperaturen zwischen 20 °C und 90 °C und ganz besonders bevorzugt Temperaturen zwischen 70 °C und 90 °C.

Die Isolierung des Produktes erfolgt nach üblichen Aufarbeitungsverfahren über das 4,5-Dichlor-2-methyl-Alkali- oder -Erdalkali-benzoat. Für den sich anschließenden Reinigungsschritt auf der Stufe des vorliegenden Salzes wird bevorzugt ein pH-Bereich eingestellt, der es erlaubt, isomere Dichlormethylbenzoesäuren abzutrennen. Bevorzugt wird eine solche Reinigung bei einem pH-Wert zwischen 8 und 12, ganz besonders bevorzugt zwischen 9 bis 10 durchgeführt. Durch geschickte Ausnutzung der Löslichkeitseigenschaften der Salze werden besonders gute Resultate erzielt. Die Isolierung eines kristallinen 4,5-Dichlor-2-methylbenzoates ist eine bevorzugte Reinigungsmethode. Ist das Salz im vorliegenden Medium in der Kälte nur wenig löslich, so kann durch Abkühlen, z.B. auf Raumtemperatur oder auch darunter, einer bisher heißen Lösung oder Suspension eine weitgehende Abscheidung erzielt werden. Bei größerer Löslichkeit kann z. B. ein Teil des Wassers abdestilliert werden, oder es kann durch Zusatz von Fremdsalzen (Aussalzen) oder von organischen Fällungsmitteln eine weitgehende Abscheidung erzielt werden. Das Kristallisat wird dann wie üblich abgetrennt, z. B. durch Filtration oder Zentrifugation. Gewünschtenfalls kann das Produkt noch gewaschen werden.

Das so in Substanz isolierte 4,5-Dichlor-2-methyl-benzoat kann, wenn die freie Säure hergestellt werden soll, nach an sich bekannten Methoden in die Säure übergeführt werden, beispielsweise durch Eintragen in wäßrige anorganische Säuren, bevorzugt Salz- oder Schwefelsäure. Die abgeschiedene Säure kann dann wie üblich z.B. durch Filtration oder Zentrifugation abgetrennt, gewaschen und getrocknet werden.

Die nach dem erfindungsgemäßen Verfahren hergestellten Alkali- und Erdalkalimetallsalze der 4,5-Dichlor-2-methylbenzoesäure sowie die daraus gewonnene freie Säure zeichnen sich durch hohe Reingehalte > 98 % aus und eignen sich damit in besonderem Maße als Zwischenprodukte bei der Synthese von Pharmazeutika und Pflanzenschutzmitteln.

Natrium-4,5-dichlor-2-methylbenzoat und 2-Chlor-1-(4,5-dichlor-2-methylphenyl) ethanon sind bisher noch nicht bekannt und sind somit ebenfalls Gegenstand vorliegender Erfindung.

### Beispiel 1:

### a) Friedel-Crafts-Acetylierung von 3,4-Dichlortoluol mit Acetylchlorid

In einem Mehrhalskolben mit KPG-Rührer, Tropftrichter, Innenthermometer und Rückflußkühler werden 450 g 3,4-Dichlortoluol (ca. 97 %ig nach GC, entsprechend 2,7 mol) vorgelegt. Unter Rühren wird zu dieser Lösung portionsweise über ca. 15 min 161,5 g wasserfreies Aluminiumchlorid (1,21 mol) zugefügt. Anschließend wird der Ansatz auf eine Innentemperatur von 42-43 °C geheizt und sodann über ca. 45-60 min 90 g Acetylchlorid (1,15 mol) so zudosiert, daß die Innentemperatur 50 °C nicht übersteigt. Nach dem Acetylchlorid-Einlauf wird ca. 4 h bei 50 °C nachgerührt. Hydrolyse:
In einem Mehrhalskolben mit KPG-Rührer und Innenthermometer werden 500 g Eis, 250 ml Wasser und 71 g konz. 36 %ige Salzsäure unter Rühren vorgelegt. In diese Eislösung wird vorsichtig der auf Raumtemperatur abgekühlte Ansatz dosiert. Man spült mit 125 g 3,4-Dichlortoluol nach und rührt den Hydrolyse-Ansatz 30 min nach.

Die organische Phase wird sodann geschieden. Anschließend wird über eine Destillationsbrücke mit Füllkörperkolonne (20 cm Höhe, Füllung: 6 mm Raschigringe) das Edukt 3,4-Dichlortoluol i. Vak. bei 20 mbar und 83 °C Kopftemperatur abdestilliert. Die Produktdestillation erfolgt i. Vak. bei 20 mbar und 125 °C Kopftemperatur.

Man erhält 387 g 3,4-Dichlortoluol (97 %ig nach GC, entsprechend 2,4 mol, entsprechend 67 % vom Gesamteinsatz), welches einem Folgeansatz direkt zugeschlagen wird.

Man erhält weiterhin 210 g (4,5-Dichlor-2-methylphenyl)ethanon in Form eines Isomerengemisches mit ca. 92 : 8 % (nach GC) bzgl. des gewünschten Isomers (entsprechend 1,04 mol tel quel, entsprechend 30 Mol-% bezogen auf den Dichlortoluoleinsatz). Dies entspricht einer Gesamtausbeute von 97 % d. Th.
- Schmp.:: 49 °C (Isomerengemisch), nach 2-fachem Umkristallisieren erreicht der Schmp.: 55-57 °*C* für isomerenreines Produkt (Ethanol)
- ¹H-NMR (300 MHz, [D₆]DMSO):: 2.4 ppm (CH₃C=, 3H), 2.6 (CH₃C=, 3H), 7.6 (H ar., 1H), 8,0 (H ar., 1H).
- ¹³C-NMR (75.5 MHz, [D₆]DMSO):: 19.9 ppm (CH₃), 29.7 (CH₃), 128,3 (-C=), 131.0 (HC=), 133.2 (HC=), 133.7 (-C=), 137.6 (-C=), 138.1 (-C=), 199.7 (-C=O).
- GC-MS (m/z):: 202, 204 (27%, 20 %) [M⁺], 187, 189, 191 (100%, 68 %, 6 %) [M⁺ - CH₃].

### b) Oxidation von (4,5-Dichlor-2-methylphenyl)ethanon

In einem 4l-Mehrhalskolben mit Zupump für Chlorbleichlauge, KPG-Rührer, Thermometer, Meßstelle für R_{H}-Messung und pH-Elektrode werden 203 g (4,5-Dichlor-2-methylphenyl)ethanon (1 mol tel quel, Isomerengemisch aus dem Verfahren gemäß a)) vorgelegt. Der Ansatz wird mit 400 ml Wasser versetzt und auf 90 °C geheizt. Der pH-Wert wird mit wenig 50 %iger Natronlauge auf 9-10 gestellt.

Sodann wird bei dieser Temperatur über ca. 3-4 h 1520 ml Chlorbleichlauge (nach Titration 145 g aktives Chlor/l, entsprechend 3,1 mol) eindosiert. Am Ende wird der NaOCl-Überschuß mit wenig Natriumpyrosulfit zerstört und der Ansatz auf 10 °C abgekühlt, wobei das Natriumsalz feinkristallin ausfällt. Es wird abfiltriert, mit 240 g ges. Kochsalz-Lösung (wäßr.) gewaschen. Man erhält so ca. 270 g wasserfeuchtes Na-Salz, welches für analytische Zwecke getrocknet werden kann.
- Schmp.:: 237-240 °C
- HPLC:: 98,0 % Reingehalt
- ¹H-NMR (300 MHz, [D₆]DMSO):: 2.4 ppm (CH₃, 3H), 7.4 (H ar., 1H), 7.8 (H ar., 1H).
- ¹³C-NMR (75.5 MHz, [D₆]DMSO):: 20.1 ppm (CH₃), 126.9 (-C=), 129.0 (-C=), 130.5 (HC=), 131.6 (HC=), 137.2 (-C=), 141.7 (-C=), 170.0 (-C=O).
- IR (KBr):: 3392 cm⁻¹ (OH/H₂O, ss), 1700 (COOH, m), 1596 (C=C, s), 1568 (COO⁻, ss), 1396 (COO⁻, ss).

Durch Einrühren von ca. 270 g Na-Salz der 4,5-Dichlor-2-methylbenzoesäure in 500 ml Wasser und 80 g konz. 36 %ige Salzsäure, Abfiltrieren, Waschen mit 250 ml Wasser und Trocknen i. Vak. wird die freie Säure gewonnen.
Man erhält 178 g 4,5-Dichlor-2-methylbenzoesäure in Form eines weißen Pulvers.

Dies entspricht einer Ausbeute von 94.2 % d. Th., bezogen auf das gewünschte Isomer.
- Schmp.:: 177-179 °C
- HPLC:: 98.4 % Reingehalt
- ¹H-NMR (300 MHz, [D₆]DMSO):: 2.5 ppm (CH₃, 3H), 7.6 (H ar., 1H), 8.0 (H ar., 1H), COO*H* verdeckt.

### Beispiel 2:

### a) Chloracetylierung von 3,4-Dichlortoluol

In einem Mehrhalskolben mit KPG-Rührer, Tropftrichter, Innenthermometer und Rückflußkühler werden 645 g 3,4-Dichlortoluol (ca. 97 %ig nach GC, entsprechend 4,0 mol) vorgelegt. Unter Rühren wird zum Edukt über ca. 15 min 161,5 g Aluminiumchlorid (1.21 mol) zugefügt. Anschließend wird in die Suspension bei 22-25 °C 124,3 g Chloracetylchlorid (98 %ig, entsprechend 1.08 mol) so dosiert, daß die Temp. 30 °C nicht übersteigt. Nach dem Chloracetylchlorid-Einlauf wird 4 h bei 40 °C nachgerührt.
Hydrolyse:
In einem Mehrhalskolben mit Rührer und Thermometer werden 600 g Eis und 60 ml konz. 36 %ige Salzsäure vorgelegt und der obige Ansatz vorsichtig eindosiert. Man spült mit 100 ml 1N Salzsäure nach. Sodann wird 3,4-Dichlortoluol über einen Wasserabscheider kontinuierlich ausgekreist, wobei die Kopftemperatur 106-108 °C beträgt. Nach Ende der Dichlortoluolauskreisung wird der Ansatz unter Rühren auf 15 °C abgekühlt, das dabei granulierte Produkt abgesaugt, mit 500 ml Wasser gewaschen und bei 45 °C i. Vak. getrocknet.

Man erhält 460 g wasserdampfdestilliertes 3,4-Dichlortoluol zurück (93 %ig nach GC, entsprechend 2,74 mol, entsprechend 70 % vom Gesamteinsatz, welches 4 GC-% Produkt enthält, entsprechend 2 Mol-% vom Gesamteinsatz), das einem Folgeansatz direkt zugeschlagen werden kann.

Man erhält weiterhin 235 g 2-Chlor-1-(4,5-dichlor-2-methylphenyl)ethanon in Form eines Isomerengemisches mit 93,5 : 6,5 (nach GC) bzgl. des gewünschten Isomers (entsprechend 1,01 mol tel quel, entsprechend 24 Mol-% bezogen auf den Dichlortoluoleinsatz). Dies entspricht einer Gesamtausbeute von 96 % d. Th.
- Schmp::: 75-77 °C (Isomerengemisch)
- ¹H-NMR (300 MHz, [D₆]DMSO]:: 2.2 ppm (CH₃C=, 0.1 H), 2.4 (CH₃C=, 3.1 H), 4.5 (CH₂, 0.2 H), 5.2 (CH₂Cl, 1.9 H), 7.6 (H ar., 0.1 H), 7.6 (H ar., 0.9 H), 8.0 (H ar., 0.1 H), 8.2 (H ar., 0.9 H).
- ¹³C-NMR (75.5 MHz, [D₆]DMSO]:: 19.5 ppm (CH₃), 49.1 (CH₂), 128.4 (-C=), 130.5 (HC=), 133.3 (HC=), 134.4 (-C=), 135.1 (-C=), 138.8 (-C=), 193.3 (C=O).
- IR (KBr):: 2991, 2956, 2934 cm⁻¹ (arom., aliph. CH, s), 1703 (C=O, ss), 1540 (C=C, ss), 1215 (CO-CH₂, ss).
- GC-MS (m/z):: 236, 238, 240 (9%, 7 %, 2 %) [M⁺], 187, 189, 191 (100 %, 62 %, 14 %) [M⁺ - CH₂Cl], 159, 161, 163 (42 %, 24 %, 6 %) [M⁺ - CH₂Cl -CO].

### b) Oxidation von 2-Chlor-1-(4,5-dichlor-2-methylphenyl)ethanon

Entsprechend Beispiel 1b) werden 237 g (1 mol tel quel) eines Isomerengemisches gemäß a) in 200 ml Wasser mit 1050 ml Chlorbleichlauge (145 g aktives Chlor/l) bei 70 °C oxidiert. Die Isolierung erfolgt über das Na-Salz. Durch Einrühren des Salzes in Salzsäure wird sodann die Säure in freier Form gewonnen. Nach dem Trocknen erhält man so 138,2 g 4,5-Dichlor-2-methylbenzoesäure mit hoher Isomerenreinheit, entsprechend 75.2 % d. Th.
- Schmp.:: 176-178 °C.

## Patentansprüche

1. Verfahren zur Herstellung von 4,5-Dichlor-2-methylbenzoesäure oder deren Salze der allgemeinen Formel I, 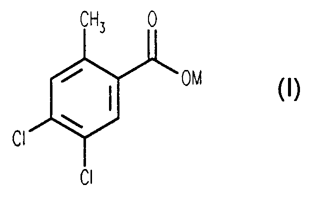 worin M für Wasserstoff oder ein Äquivalent eines Alkali- oder Erdalkalimetalls steht, dadurch gekennzeichnet, daß man 3,4-Dichlortoluol in Gegenwart eines Friedel-Crafts-Katalysators acetyliert oder Halogen-acetyliert, das erhaltene Isomerengemisch in einer Haloformreaktion in Gegenwart eines Alkali- oder Erdalkalihydroxids oxidiert, anschließend die erhaltene 4,5-Dichlor-2-methylbenzoesäure in Form eines ihrer Alkali- oder Erdalkalisalzes reinigt und gegebenenfalls das Salz in die freie Saure überführt.

2. Verfahren gemäß Anspruch 1, dadurch gekennzeichnet, daß man in überschüssigem 3,4-Dichlortoluol arbeitet.

3. Verfahren gemäß Anspruch 1 und/oder 2, dadurch gekennzeichnet, daß man als Acetylierungsmittel Acetylchlorid, Chloracetylchlorid oder Acetanhydrid einsetzt.

4. Verfahren gemäß einem oder mehreren der Ansprüche 1 bis 3, dadurch gekennzeichnet, daß man als Friedel-Crafts-Katalysator Aluminiumchlorid einsetzt.

5. Verfahren gemäß einem oder mehreren der Ansprüche 1 bis 4, dadurch gekennzeichnet, daß man die Acetylierung bei Temperaturen von 10 bis 90 °C durchführt.

6. Verfahren gemäß einem oder mehreren der Ansprüche 1 bis 5, dadurch gekennzeichnet, daß als Alkali- oder Erdalkalihydroxid Lithium-, Natrium-, Kalium-, Rubidium-, Cäsium-, Magnesium-, Calcium-, Strontium- oder Bariumhydroxid eingesetzt wird.

7. Verfahren gemäß einem oder mehreren der Ansprüche 1 bis 6, dadurch gekennzeichnet, daß als Oxidationsmittel Hypochlorit, Hypobromit oder Hypoiodit eingesetzt werden.

8. Verfahren gemäß einem oder mehreren der Ansprüche 1 bis 7, dadurch gekennzeichnet, daß die 4,5-Dichlor-2-methylbenzoesäure in Form eines ihrer Alkali- oder Erdalkalisalze durch Kristallisation bei einem pH-Wert zwischen 8 und 12 gereinigt wird.

9. Natrium-4,5-dichlor-2-methylbenzoat.

10. 2-Chlor-1-(4,5-dichlor-2-methylphenyl)ethanon.
